# EUROPÄISCHE PATENTSCHRIFT

(11) **EP 1 163 015 B1**
(45) Veröffentlichungstag und Bekanntmachung des Hinweises auf die Patenterteilung: **06.10.2004**
(21) Anmeldenummer: 00912528.7
(22) Anmeldetag: 01.03.2000
(51) Int. Cl.: A61L 15/28

(54) **STILLEINLAGE**
BREAST-MILK ABSORBENT PAD
COUSSINET D'ALLAITEMENT

(30) Priorität: 25.03.1999 DE 19913478
(43) Veröffentlichungstag der Anmeldung: 19.12.2001
(73) Patentinhaber: Paul Hartmann Aktiengesellschaft, 89522 Heidenheim (DE)
(72) Erfinder: BITTERHOF, Andreas, D-89564 Nattheim-Auernheim (DE)
(74) Vertreter: Friz, Oliver
(86) Internationale Anmeldenummer: PCT/EP2000/001716
(87) Internationale Veröffentlichungsnummer: WO 2000/057931

(56) Entgegenhaltungen:
- EP-A- 0 816 405
- EP-A- 0 844 270
- WO-A-92/16681
- GB-A- 977 708
- GB-A- 2 292 526
- PATENT ABSTRACTS OF JAPAN vol. 017, no. 478 (C-1104), 31. August 1993 (1993-08-31) & JP 05 117431 A (SNOW BRAND MILK PROD CO LTD), 14. Mai 1993 (1993-05-14)

## Beschreibung

Die Erfindung betrifft eine Stilleinlage mit einer Flüssigkeit aufnehmenden Saugschicht, die üblicherweise aus einem Fasermaterial gebildet ist und gegebenenfalls superabsorbierende Hydrogelmaterialien zur Aufnahme und Bindung von Flüssigkeit aufweisen kann. Eine derartige Stilleinlage ist beispielsweise in EP 0 698 385 A2 beschrieben.

Stilleinlagen werden verwendet, um bei stillenden Müttern zwischen den Stillphasen nachtropfende Muttermilch aufzunehmen und zu verhindern, dass hierdurch Bekleidungsstücke verunreinigt werden. Sie dienen daher auch der Diskretion. Ferner sind Stilleinlagen üblicherweise mit einer als hautverträglich und weich empfundenen körperzugewandten Innenseite ausgestattet, um keine Hautirritationen hervorzurufen, wie dies möglicherweise ein Bekleidungsstück an der durch das Stillen strapazierten Brust auslösen könnte.

Muttermilch besteht zu einem Anteil von 2 bis 8 Masse %, im Mittel zu 4,5 Masse %, aus Fett. Die in Stilleinlagen üblicherweise eingesetzten Materialien, nämlich Fasermaterial auf der Basis von Zellstoff und gegebenenfalls superabsorbierende Hydrogelmaterialien, absorbieren nur den wässrigen Teil der nachtropfenden Muttermilch. Die Fettphase bleibt unabsorbiert; dies bedeutet, sie haftet an Oberflächen der absorbierenden Materialien an. Hierdurch wird die aktive, zur Flüssigkeitsaufnahme zur Verfügung stehende Oberfläche verringert und die Wirksamkeit des Absorptionsprozesses gestört bzw. herabgesetzt. Dies kann dazu führen, dass nach einer gewissen Zeit und in Abhängigkeit der nachtropfenden Muttermilch diese nicht mehr wirksam absorbiert werden kann, sondern nach unten rinnt.

Hiervon ausgehend liegt der vorliegenden Erfindung die Aufgabe zugrunde, eine Stilleinlage der eingangs genannten Art so zu verbessern, dass die vorstehend beschriebenen Nachteile überwunden werden können und die an sich zur Verfügung stehende Flüssigkeitsaufnahmekapazität der Stilleinlage besser ausgenützt werden kann.

Diese Aufgabe wird durch eine Stilleinlage der genannten Art erfindungsgemäss dadurch gelöst, dass sie zusätzlich Chitosan umfasst.

Chitosan ist ein Naturstoff, und zwar ein Biopolymer, das aus Chitin, der Schale von Krustentieren, insbesondere durch Deacetilierung gewonnen werden kann. Es kann in Form eines Pulvers oder in Faserform hergestellt und in die Stilleinlage eingebracht werden.

Aus EP-A-0 844 270 ist Chitosan als Füllstoff für Formpreßteile bekannt; eine Anwendung von Chitosan zur Fettabsorption ist nicht offenbart.

Es ist bekannt, dass Chitosan als wirksamer Fettabsorber eingesetzt werden kann (D. Henrichs, Handbuch "Nähr- und Vitalstoffe", MZ-Verlag). Darüber hinaus ist es bekannt, Chitosanderivate in einer schier unüberschaubaren Vielfalt herzustellen und diese als Adsorbens, beipielsweise für Schwermetalle, einzusetzen, was in der JP 61-133143-A beschrieben ist.

Die DE 43 18 094 A1 lehrt die Verwendung von mit Säure umgesetztem Chitosan als superabsorbierendes Mittel in einem Hygieneartikel. Im Vordergrund steht das Absorptionsvermögen von Urin.

In ganz besonders vorteilhafter Weise ist Chitosan als in der Natur vorkommendes Biopolymer auf natürliche Weise abbaubar. Werden die übrigen Komponenten der Stilleinlage in Form einer beim Gebrauch körperabgewandten Aussenlage und der beim Gebrauch körperzugewandten Lage aus biologisch abbaubaren Materialien ausgebildet, so kann die Stilleinlage als kompostierbares Produkt angeboten werden. Für die körperzugewandte flüssigkeitsdurchlässige Lage kann vorzugsweise ein Viskosevliesmaterial und für die Aussenlage ein Vliesstoff oder eine Folie auf der Basis von Viskose, PVA (Polyvinylalkohol), Polyesteramid, Stärkecopolyester, Stärke/Polycaprolactam-Mischung, Copolymer aus Polyhydroxybuttersäuere (PHB) und Polyhydroxyvaleriansäure (PLA) gewählt werden. Die Außenlage kann auch ein hydrophober Vliesstoff, beispielsweise Polypropylen, sein.

Das Chitosan kann auch in der körperzugewandten Lage, also in unmittelbarem Körperkontakt, angeordnet sein. Es erweist sich indessen als vorteilhaft, wenn das Chitosan in der eigentlichen Saugschicht angeordnet ist. Diese Saugschicht ist vorteilhafterweise aus 1 - 5 g, vorzugsweise aus 2 - 4 g, auf Zellstoffbasis hergestelltem Fluff gebildet. Sie kann auch superabsorbierende Polymermaterialien umfassen. Solchenfalls ist die Saugschicht vorteilhafterweise aus 1,5 - 2,5 g Fluff und 0,5 - 1,5 g superabsorbierendem Polymermaterial gebildet.

Es hat sich als vorteilhaft und hinreichend erwiesen, wenn 0,2 - 1 g Chitosan je Stilleinlage verwendet sind.

Der masseprozentuale Anteil von Chitosan in der Saugschicht beträgt vorteilhafterweise zwischen 5 und 25 %.

## Patentansprüche

1. Stilleinlage mit einer Flüssigkeit aufnehmenden Saugschicht aus einem Fasermaterial, gegebenenfalls auch superabsorbierende Polymermaterialien enthaltend, **dadurch gekennzeichnet, dass** die Saugschicht zusätzlich 0,2 - 1 g Chitosan enthält.

2. Stilleinlage nach Anspruch 1, **dadurch gekennzeichnet, dass** die Saugschicht 1 - 5 g, insbesondere 2 - 4 g, Fluff aufweist.

3. Stilleinlage nach Anspruch 2, **dadurch gekennzeichnet, dass** die Saugschicht 1,5 - 2,5 g Fluff und 0,5 - 1,5 g superabsorbierende Polymermaterialien umfasst.

4. Stilleinlage nach Anspruch 1, 2 oder 3, **dadurch gekennzeichnet, dass** der masseprozentuale Anteil des Chitosans in der Saugschicht 5 - 25 Masse-% beträgt.

5. Stilleinlage nach einem der vorstehenden Ansprüche, **gekennzeichnet durch** eine beim Gebrauch körperabgewandte Außenlage in Form eines hydrophoben Vliessstoffs oder in Form einer Folie oder eines Vlies-Folien-Laminats.

6. Stilleinlage nach einem der vorstehenden Ansprüche, **gekennzeichnet durch** eine beim Gebrauch körperzugewandte flüssigkeitsdurchlässige Lage aus Viskose oder Viskose/Polyester.

7. Stilleinlage nach einem der vorstehenden Ansprüche, **dadurch gekennzeichnet, dass** sie nur aus vollständig biologisch abbaubaren Materialien gebildet ist.

## Claims

1. Breast-milk absorbent pad having a fluid-absorbent layer formed of a fibre material, optionally also containing superabsorbent polymer materials, **characterised in that** the absorbent layer additionally contains 0.2 to 1 g of chitosan.

2. Breast-milk absorbent pad according to claim 1, **characterised in that** the absorbent layer contains 1 to 5 g, in particular 2 to 4 g, of fluffy material.

3. Breast-milk absorbent pad according to claim 2, **characterised in that** the absorbent layer comprises 1.5 to 2.5 g of fluffy material and 0.5 to 1.5 g of superabsorbent polymer materials.

4. Breast-milk absorbent pad according to claim 1, 2 or 3, **characterised in that** the percentage by mass of the chitosan in the absorbent layer amounts to 5 to 25 %.

5. Breast-milk absorbent pad according to any one of the preceding claims, **characterised by** an external layer in the form of one of a hydrophobic nonwoven material, or in the form of a film or a nonwoven-film laminate, facing away from a body in use.

6. Breast-milk absorbent pad according to any one of the preceding claims, **characterised by** a fluid-permeable layer of viscose or viscose/polyester facing a body in use.

7. Breast-milk absorbent pad according to any one of the preceding claims, **characterised in that** it is formed only of completely biodegradable materials.

## Revendications

1. Coussinet d'allaitement comportant une couche absorbante en matière fibreuse qui absorbe le liquide, contenant aussi le cas échéant des matières polymères ultra-absorbantes, **caractérisé en ce que** la couche absorbante contient en plus 0,2 à 1 g de chitosan.

2. Coussinet d'allaitement selon la revendication 1, **caractérisé en ce que** la couche absorbante présente 1 à 5 g, en particulier 2 à 4 g, de bourre.

3. Coussinet d'allaitement selon la revendication 2, **caractérisé en ce que** la couche absorbante contient 1,5 à 2,5 g de bourre et 0,5 à 1,5 g de matières polymères ultra-absorbantes.

4. Coussinet d'allaitement selon la revendication 1, 2 ou 3, **caractérisé en ce que** le pourcentage en masse de chitosan dans la couche absorbante est compris entre 5 et 25 % en masse.

5. Coussinet d'allaitement selon l'une des revendications précédentes, **caractérisé par** une couche extérieure orientée à l'opposé du corps en utilisation réalisée sous la forme d'une étoffe nappée hydrophobe ou sous la forme d'un film ou d'un laminé non-tissé/film.

6. Coussinet d'allaitement selon l'une des revendications précédentes, **caractérisé par** une couche perméable aux liquides en viscose ou viscose/polyester orientée vers le corps en utilisation.

7. Coussinet d'allaitement selon l'une des revendications précédentes, **caractérisé en ce qu'**il est réalisé uniquement à partir de matières entièrement biodégradables.
